Europäisches Patentamt

European Patent Office

Office européen des brevets

(19)

(11) Veröffentlichungsnummer: **0 173 219**
**B1**

(12) # EUROPÄISCHE PATENTSCHRIFT

(45) Veröffentlichungstag der Patentschrift:
**11.05.88**

(21) Anmeldenummer: **85110415.8**

(22) Anmeldetag: **20.08.85**

(51) Int. Cl.⁴: **C 07 C 45/50,** C 07 C 47/02

(54) **Verfahren zur Herstellung von Aldehyden.**

(30) Priorität: **30.08.84 DE 3431840**

(43) Veröffentlichungstag der Anmeldung:
**05.03.86 Patentblatt 86/10**

(45) Bekanntmachung des Hinweises auf die Patenterteilung:
**11.05.88 Patentblatt 88/19**

(84) Benannte Vertragsstaaten:
**AT BE DE FR GB IT LU NL SE**

(56) Entgegenhaltungen:
**AT - B - 371 431**
**DE - A - 3 234 701**
**DE - B - 2 627 354**

**Die Akte enthält technische Angaben, die nach dem Eingang der Anmeldung eingereicht wurden und die nicht in dieser Patentschrift enthalten sind.**

(73) Patentinhaber: **Ruhrchemie Aktiengesellschaft, Bruchstrasse 219, D-4200 Oberhausen 11 (DE)**

(72) Erfinder: **Cornils, Boy, Dr. Dipl.-Chem., Friedrich-Ebert-Strasse 45, D-4220 Dinslaken (DE)**
Erfinder: **Bahrmann, Helmut, Dr. Dipl.-Chem., Rohstrasse 48, D-4236 Hamminkeln-Brünen (DE)**
Erfinder: **Lipps, Wolfgang, Dr. Dipl.-Chem., Kieningstrasse 6, D-7972 Isny/Allgäu (DE)**
Erfinder: **Konkol, Werner, Dr. Dipl.-Chem., Lützowstrasse 40 a, D-4200 Oberhausen 11 (DE)**

(74) Vertreter: **Reichelt, Karl-Heinz, Dr., Ruhrchemie Aktiengesellschaft Abt. PLD Postfach 13 01 60, D-4200 Oberhausen 11 (DE)**

**Beschreibung**

Die vorliegende Erfindung betrifft ein Verfahren zur Herstellung von Aldehyden durch Hydroformylierung von Olefinen in Gegenwart wasserlöslicher Rhodium-Komplexkatalysatoren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in grossem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Masse iso-Aldehyde gebildet. Schliesslich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form modifizierter Hydridorhodiumcarbonyle eingesetzt, die zusätzliche Liganden, die auch im Überschuss vorhanden sein können, enthalten. Besonders bewährt haben sich als Liganden tertiäre Phosphine oder Phosphite. Ihre Anwendung ermöglicht, den Reaktionsdruck auf Werke unter 300 bar (30 MPa) zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen im Molekül, beschritten werden. Ausserdem hat sich gezeigt, dass die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind.

Derartige Katalysatoren sind z.B. in der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wässriger und organischer Phase, d.h. ohne Destillation und damit ohne zusätzliche thermische Verfahrensschritte. Neben sulfonierten Triarylphosphinen werden als Komplexbestandteile wasserlöslicher Rhodiumkomplexverbindungen auch carboxylierte Triarylphosphine eingesetzt.

Die DE-A1-3 234 701 betrifft ein Verfahren zur Hydroformylierung von Olefinen bei erhöhter Temperatur und erhöhtem Druck unter Verwendung eines in der DE-PS 26 27 354 beschriebenen Katalysatorsystems. Der Anteil der gasförmigen Bestandteile in der flüssigen Phase beträgt 5 bis 30 Vol.-%, bezogen auf die Mischphase.

Die Reaktion des Olefins mit Kohlenmonoxid und Wasserstoff läuft in der wässrigen, den Katalysator enthaltenden Phase ab.

Während bei niedrigen Olefinen wie Ethylen und Propylen der Umsatz so hoch wie bei den bekannten Verfahren ist, fällt er bei Verwendung höherer Olefine merklich ab, so dass beispielsweise 1-Hexen oder 1-Decen nur in unbefriedigendem Masse reagieren.

Verursacht wird diese Umsatzminderung vermutlich dadurch, dass die Löslichkeit und damit die Konzentration der Olefine in der wässrigen Phase mit steigender Zahl der Kohlenstoffatome abnimmt. Eine Erhöhung der Rührintensität zur besseren Verteilung des Olefins in der wässrigen Phase bringt nur eine geringe Erhöhung des Umsatzes.

In Nachr. Chem. Tech. Lab. 31 (1983) 10, 798 ff wird über die Anwendung von Ultraschall in der organischen Synthese berichtet. Ultraschall beschleunigt unter anderem heterogene Reaktionen, insbesondere solche, an denen Feststoffe und Flüssigkeiten beteiligt sind. Als Beispiele werden neben anderen Umsetzungen die partielle Reduktion von symmetrischen oder unsymmetrischen alpha,alpha-Dibromketonen mit Hilfe von Quecksilber, die 2-Phasen-Verseifung von Estern, die Bildung von Organolithium- und Grignardverbindungen und die Synthese von Lithiumorganocupraten genannt. Über die Anwendung von Ultraschall auf Dreiphasensysteme an denen auch eine gasförmige Phase beteiligt ist, wird nicht berichtet.

Es bestand die Aufgabe, ein Verfahren zu entwickeln, das die Bildung von Aldehyden aus Olefinen, insbesondere solche mit bis zu 20 Kohlenstoffatomen, vorzugsweise mit 4-20 Kohlenstoffatomen, durch Hydroformylierung erlaubt, wobei ein hoher Umsatz des Ausgangsolefins je Zeiteinheit bei gegebener Katalysatormenge zum gewünschten Endprodukt sichergestellt wird.

Die vorstehend skizzierte Aufgabe wird gelöst durch ein Verfahren zur Herstellung von Aldehyden durch Umsetzung von Olefinen mit Kohlenmonoxid und Wasserstoff in Gegenwart von Wasser und einer wasserlöslichen Rhodium enthaltenden Komplexverbindung als Katalysator unter erhöhtem Druck und unter erhöhter Temperatur, dadurch gekennzeichnet, dass man bei einer Temperatur von 20 bis 160°C und unter einem Druck von 0,1 bis 10 MPa Olefine mit 2 bis 20 Kohlenstoffatomen mit einer wässrigen Phase, die je kg 0,1 bis 15 mmol der wasserlöslichen Rhodiumkomplexverbindung enthält, umsetzt, das Volumverhältnis von wässriger Phase zu organischer Phase 1:100 bis 100:1 beträgt, auf das Reaktionsgemisch Ultraschall einwirkt und man gegebenenfalls rührt.

Obgleich durch Einwirkung von Ultraschall auf das aus flüssiger organischer und wässriger und zusätzlich noch gasförmiger Phase bestehende Reaktionssystem eine intensive Durchmischung der flüssigen Reaktanten erreicht wird, war nicht vorauszusehen, dass eine Erhöhung des Umsatzes eintritt, denn es ist bekannt, dass Ultraschall die Löslichkeit von Gasen in Flüssigkeiten herabsetzt und die Entgasung von Flüssigkeiten beschleunigt. Darüber hinaus musste befürchtet werden, dass der Katalysator der Einwirkung von Ultraschall nicht standhält. Hierbei ist zu berücksichtigen, dass durch den Ultraschall in Mikrobereichen Stosswellen auftreten, die zu sehr hohen Drücken und Temperaturen führen können. Berücksichtigt man, dass bereits die thermische Belastung bei der Destillation zur Zersetzung des Katalysators führt, war zu erwarten, dass auch Ultraschall eine Zerstörung des Katalysators herbeiführt.

Die Umsetzung der Ausgangsstoffe erfolgt in einem mit Rührvorrichtung ausgerüsteten Druckreaktor aus Metall oder Glas. Ihm werden die Reaktanten, d.h. Olefin und Synthesegas, sowie die wässrige Katalysatorlösung gemeinsam oder getrennt, zugeführt. Der Einsatz von Verteilungsvorrichtungen wie Siebböden oder Fritten für die gasförmigen Komponenten hat sich bewährt. Es ist auch möglich, Rührung und Verteilung der gasförmigen Reaktionspartner miteinander zu kombinieren, z.B. durch Verwendung eines Begasungsrührers.

Unter Ultraschall versteht man Schallwellen einer Frequenz von 20 und mehr kHz. Bevorzugt verwendet man Ultraschallsender, die eine Frequenz von 20 bis 60 kHz ausstrahlen. Es ist jedoch möglich, auch höhere Frequenzen, beispielsweise 200 kHz mit gutem Erfolg zu verwenden.

Als Ultraschallsender können handelsübliche Ultraschallreinigungsgeräte verwendet werden. Als Reaktoren eignen sich in diesem Fall auch Glasgefässe. Die Übertragung der Schallenergie erfolgt hierbei durch Vermittlung niedrigviskoser Flüssigkeiten. Diese Art der Verfahrensführung ist jedoch auf Umsetzungen bei niedrigen Drücken beschränkt.

Zur Durchführung der Reaktion bei höheren Drücken setzt man entsprechend dimensionierte Ultraschalltauchschwinger direkt in das Reaktionsgefäss ein. Diese Arbeitsweise hat den Vorteil, dass die Schallenergie unmittelbar auf das Reaktionsgemisch einwirkt, so dass Leistungsverluste, wie bei der Verwendung von Überträgerflüssigkeiten nicht auftreten. Tauchschwinger können sowohl in Rührautoklaven und grösseren Rührreaktoren als auch in Strömungsrohren eingeführt werden.

Die erfindungsgemäss verwendeten wasserlöslichen Katalysatoren sind Komplexverbindungen des Rhodiums, die neben Kohlenmonoxid und Wasserstoff sulfonierte oder carboxylierte Phosphine enthalten. Bevorzugt sind sulfonierte oder carboxylierte Triarylphosphine, insbesondere Triphenylphosphine oder Trinaphthylphosphine. Es ist nicht erforderlich, dass alle drei Arylreste Sulfonsäuregruppen oder Carboxylgruppen tragen. Es hat sich gezeigt, dass bereits eine Sulfonsäuregruppe oder eine Carboxylgruppe im Phosphinmolekül der Komplexverbindung eine ausreichende Wasserlöslichkeit verleiht. Der Katalysator kann dem Reaktionsgemisch präformiert zugesetzt werden. Es ist aber auch möglich, ihn in situ zu bilden. Besonders bewährt hat es sich, die wasserlöslichen Phosphine im Überschuss einzusetzen. Bevorzugt wendet man je g-Atom-Rhodium 5 bis 100 mol des Phosphins an.

Die Konzentration der als Katalysator eingesetzten Rhodium-Komplex-Verbindung in der wässrigen Phase beträgt 0,1-15 mmol und vorzugsweise 0,8-12 mmol je kg wässriger Phase.

Das Volumverhältnis von wässriger zu organischer Phase ist 1:100 bis 100:1. Besonders bewährt hat sich ein Volumverhältnis von 10:1 bis 100:1.

Die Umsetzung der Reaktanten erfolgt bei 20-160°C, bevorzugt 80-140°C unter Drücken von 0,1 bis 10 MPa, vorzugsweise 1-5 MPa.

Das für die Hydroformylierung verwendete Synthesegas enthält Kohlenmonoxid und Wasserstoff zweckmässigerweise im Volumverhältnis 1:1. Es ist aber auch möglich, dieses Verhältnis zu variieren und Gasgemische einzusetzen, die kohlenmonoxid- oder wasserstoffreicher sind.

Die neue Arbeitsweise eignet sich besonders zur Umsetzung von Olefinen mit 2-20 Kohlenstoffatomen zu den um ein Kohlenstoffatom reicheren Aldehyden. Beispiele für geeignete Olefine sind Buten, Penten, Hexen, Diisobutylen, Tripropylen, Decen, Dicyclopentadien, Undecen, Dodecen, Tetrapropylen, Pinen, Limonen, Terpinen, Camphen, Ölsäure, Ölsäureester, Elaidinsäure und Elaidinsäureester.

Nachstehend wird die Erfindung durch eine Reihe Beispiele beschrieben. Als Mass für den Umsatz wird die Turn-Over-Number (TON) verwendet. Sie ist definiert durch den Ausdruck

$$\text{TON} = \frac{\text{mmol Aldehyd}}{\text{mg-Atom Rh} \times t} \left[ \frac{1}{\text{min}} \right]$$

Die Abkürzung TPPTS steht für das Natriumsalz des Triphenylphosphintrisulfonats.

### Beispiel 1

In ein Glasgefäss werden 0,218 mmol (400 mg) HRh(CO)(TPPTS)$_3$, gelöst in 200 ml Wasser und 200 g n-Hexen-1 gegeben. Durch das intensiv gerührte Gemisch werden bei 30°C und unter Normaldruck während 7 Stunden 60 l/h Synthesegas (CO : H$_2$ = 1 : 1) geleitet. Nach gaschromatographischer Analyse erhält man 6,29 mmol n-Heptanal und 1,11 mmol 2-Methylhexanal. Die TON beträgt 0,08 min$^{-1}$.

### Beispiel 2

Die nach Abtrennung der organischen Phase zurückerhaltene wässrige Katalysatorlösung des Beispiels 1 wird mit 200 g n-Hexen-1 versetzt. Unter den Bedingungen des Beispiels 1, jedoch bei zusätzlicher Einwirkung von Ultraschall einer Frequenz von 35 kHz, leitet man durch das Gemisch während 15 Stunden 60 l/h Synthesegas (CO : H$_2$ = 1 : 1). Nach gaschromatographischer Analyse erhält man 30,6 mmol n-Heptanal und 5,4 mmol 2-Methylhexanal. Die TON beträgt 0,183 min$^{-1}$. Sie ist damit gegenüber Beispiel 1 um das 2,3 fache gestiegen.

### Beispiel 3 (Vergleichsversuch)

In einem Glasautoklaven, der mit Rührer, Temperaturmessvorrichtung und Probenahmestutzen versehen ist, werden 0,33 mmol (600 mg) HRh(CO)-(TPPTS)$_3$ gelöst in 180 g Wasser mit 200 g n-Hexen-1 versetzt. Mit Synthesegas (CO : H$_2$ = 1 : 1), das im Masse seines Verbrauches nachgeliefert wird, stellt man einen Druck von 1 MPa ein und lässt unter Rühren (Drehzahl: 500 min⁻¹) bei 35°C 3 h reagieren. Nach gaschromatographischer Analyse erhält man 30,3 mmol n-Heptanal und 7,6 mmol 2-Methylhexanal. Die TON beträgt 0,637.

### Beispiel 4

Die wässrige Katalysatorlösung aus Beispiel 3 wird mit 300 g n-Hexen-1 versetzt. Unter den Bedingungen des Beispiels 3 wird das Gemisch während 5 Stunden Reaktionszeit zusätzlich mit Ultraschall behandelt. Man erhält 115 mmol n-Heptanal und 29 mmol 2-Methylhexanal. Die TON beträgt 1,45.

### Beispiel 5 (Vergleichsversuch)

In einem 2 l-Stahlautoklaven, versehen mit Rührer, Temperaturmessvorrichtung und Probenahmestutzen befinden sich zwei handelsübliche Tauchschwinger, die 23 sowie 40 kHz Ultraschall erzeugen. Ihre Leistungsaufnahme beträgt je 300 Watt. Der Reaktor wird mit 2 mmol (3,674 g) HRh(CO)-(TPPTS)₃, gelöst in 600 g Wasser, und 600 g n-Hexen-1 beschickt. Mit Synthesegas (CO : H₂ = 1 : 1), das im Masse seines Verbrauches nachgeliefert wird, stellt man einen Druck von 1 MPa ein und lässt unter Rühren (Drehzahl 500 min⁻¹) bei 35°C ohne Einwirkung von Ultraschall 5 Stunden reagieren. Man erhält 389 mmol n-Heptanal und 97 mmol 2-Methylhexanal. Die TON beträgt 0,81.

### Beispiel 6

Das Reaktionsgemisch aus Beispiel 5 wird in der unter Beispiel 5 beschriebenen Apparatur weitere 3 Stunden zusätzlich mit Ultraschall (Leistungsaufnahme der Tauchschwinger: 2 × 300 Watt) behandelt. Mit Synthesegas (CO : H₂ = 1 : 1), das im Masse seines Verbrauches nachgeliefert wird, stellt man einen Druck von 1 MPa ein und lässt unter Rühren (Drehzahl: 500 min⁻¹) bei 35°C reagieren. Man erhält weitere 861 mmol n-Heptanal und 189 mmol 2-Methylhexanal. Die TON beträgt 2,92.

### Beispiel 7 (Vergleichsversuch)

Die wässrige Katalysatorlösung aus Beispiel 6 wird mit 600 g n-Hexen-1 versetzt. Mit Synthesegas (CO : H₂ = 1 : 1), das im Masse seines Verbrauches nachgeliefert wird, stellt man einen Druck von 2,5 MPa ein und lässt unter Rühren (Drehzahl: 500 min⁻¹) bei 35°C 3 Stunden reagieren. Man erhält 945 mmol n-Heptanal und 222 mmol 2-Methylhexanal. Die TON beträgt 3,24.

### Beispiel 8

Das Reaktionsgemisch aus Beispiel 7 wird unter den Bedingungen des Beispiels 7 während 30 Minuten zusätzlich mit Ultraschall behandelt. Es bilden sich weitere 554 mmol n-Heptanal und 136 mmol 2-Methylhexanal. Die TON beträgt 11,34.

### Beispiel 9 (Vergleichsversuch)

Die wässrige Katalysatorlösung aus Beispiel 8 wird mit 600 g n-Hexen versetzt und analog Beispiel 7, aber unter einem Druck von 5 MPa (CO : H₂ = 1 : 1), umgesetzt. Nach einer Stunde Reaktionsdauer werden 462 mmol n-Heptanal sowie 102 mmol 2-Methylhexanal erhalten. Die TON beträgt 4,70.

### Beispiel 10

Das Reaktionsgemisch aus Beispiel 9 wird unter den Bedingungen des Beispiels 9, während 30 Minuten zusätzlich mit Ultraschall behandelt. Es werden weitere 722 mmol n-Heptanal und 180 mmol 2-Methylhexanal erhalten. Die TON beträgt 5,04.

### Beispiel 11 (Vergleichsversuch)

200 g Diisobutylen und 0,330 mmol (600 g) HRh(CO)(TPPTS)₃, gelöst in 200 g Wasser, werden in einen Glasautoklaven gegeben. Mit Synthesegas (CO : H₂ = 1 : 1), das im Masse seines Verbrauches nachgeliefert wird, stellt man einen Druck von 1 MPa ein und lässt unter Rühren (Drehzahl: 400 min⁻¹) bei 35°C 4 Stunden reagieren. Die Bildung von Aldehyden lässt sich gaschromatographisch nicht nachweisen.

Unter den gleichen Bedingungen, jedoch mit auf 1 900 min⁻¹ erhöhter Drehzahl, wird das Gemisch weitere 4 Stunden umgesetzt. Man erhält 0,053 mmol C₉-Aldehyde. Die TON beträgt $6,7 \times 10^{-4}$.

### Beispiel 12

Das Reaktionsgemisch aus Beispiel 11 wird unter den Bedingungen des Beispiels 11 während 4 Stunden zusätzlich mit Ultraschall behandelt. Man erhält weitere 0,76 mmol C₉-Aldehyde. Die TON beträgt $9,6 \times 10^{-3}$.

### Präformierung einer Rh-TPPTS-Lösung mit CO/H₂

In einem 5 l-Stahlautoklaven werden 987 mmol (561 g) TPPTS, sowie 14,58 mmol (1,5 g Rh) Rhodiumacetat, gelöst in 3 kg Wasser, vorgelegt. Diese Lösung wird 3 h bei 125°C mit Synthesegas (CO : H₂ = 1 : 1) bei einem Druck von 2,5 MPa behandelt. Das P : Rh-Verhältnis beträgt 67 : 1. Die Lösung enthält 500 ppm Rhodium. Sie wird in den Beispielen 13-16 verwendet.

### Beispiel 13 (Vergleichsversuch)

180 g der präformierten Rh-TPPTS-Katalysator-Lösung und 200 g n-Hexen-1 werden in einen Glasautoklaven gegeben. Mit Synthesegas (CO : H₂ = 1 : 1), das im Masse seines Verbrauches nachgeliefert wird, stellt man einen Druck von 1 MPa ein und lässt unter Rühren bei 120°C 5 Stunden reagieren. Es werden 111,4 mmol n-Heptanal und 1,1 mmol 2-Methylhexanal erhalten. Die TON beträgt 0,43.

### Beispiel 14

Das Reaktionsgemisch aus Beispiel 13 wird unter den Bedingungen des Beispiels 13 zusätzlich mit Ultraschall behandelt. Nach 5 Stunden Reaktionsdauer erhält man weitere 279 mmol n-Heptanal und 3 mmol 2-Methylhexanal. Die TON beträgt 1,075.

### Beispiel 15 (Vergleichsversuch)

In dem Stahlautoklaven des Beispiels 5, der mit zwei Tauchschwingern bestückt ist, werden 600 g der präformierten Rh-TPPTS-Katalysatorlösung und 600 g n-Hexen-1 vorgelegt. Mit Synthesegas (CO : H₂ = 1 : 1), das im Masse seines Verbrauches nachgeliefert wird, stellt man einen Druck von 2,5 MPa ein und lässt unter Rühren bei 120°C 3 Stunden reagieren. Man erhält 114 mmol n-Heptanal und 2 mmol 2-Methylhexanal. Die TON beträgt 0,74.

*Beispiel 16*

Das Reaktionsgemisch aus Beispiel 15 wird unter den Bedingungen des Beispiels 15 während 2 Stunden zusätzlich mit Ultraschall (Leistungsaufnahme der Tauchschwinger 2 × 300 Watt) behandelt. Man erhält weitere 616 mmol n-Heptanal und 6 mmol 2-Methylhexanal. Die TON beträgt 1,78.

**Patentansprüche**

1. Verfahren zur Herstellung von Aldehyden durch Umsetzung von Olefinen mit Kohlenmonoxid und Wasserstoff in Gegenwart von Wasser und einer wasserlöslichen Rhodium enthaltenden Komplexverbindung als Katalysator unter erhöhtem Druck und unter erhöhter Temperatur, dadurch gekennzeichnet, dass man bei einer Temperatur von 20 bis 160°C und unter einem Druck von 0,1 bis 10 MPa Olefine mit 2 bis 20 Kohlenstoffatomen mit einer wässrigen Phase, die je kg 0,1 bis 15 mmol der wasserlöslichen Rhodiumkomplexverbindung enthält, umsetzt, das Volumverhältnis von wässriger Phase zu organischer Phase 1 : 100 bis 100 : 1 beträgt, auf das Reaktionsgemisch Ultraschall einwirkt und man gegebenenfalls rührt.

2. Verfahren nach Anspruch 1, dadurch gekennzeichnet, dass die Umsetzung bei Temperaturen von 80-140°C durchgeführt wird.

3. Verfahren nach Anspruch 1 und 2, dadurch gekennzeichnet, dass die Umsetzung bei Drücken von 1 bis 5 MPa durchgeführt wird.

4. Verfahren nach Anspruch 1 bis 3, dadurch gekennzeichnet, dass das Volumen-Verhältnis von wässriger zu organischer Phase 10 : 1 bis 100 : 1 beträgt.

5. Verfahren nach Anspruch 1 bis 4, dadurch gekennzeichnet, dass die Konzentration der Rhodium enthaltenden Komplexverbindung in der wässrigen Phase 0,8-12,0 mmol/kg wässriger Phase beträgt.

**Claims**

1. A process for the preparation of aldehydes by the reaction of olefins with carbon monoxide and hydrogen in the presence of water and a water-soluble rhodium-containing complex compound as a catalyst at elevated pressure and temperature, characterised in that, at a temperature of 20 to 160°C and a pressure of 0.1 to 10 MPa, olefins with 2 to 20 carbon atoms are reacted with an aqueous phase containing 0.1 to 15 mmol of the water-soluble rhodium complex compound per kg, the volume ratio of aqueous phase to organic phase is 1 : 100 to 100 : 1, the reaction mixture is subjected to ultrasonic waves and, if necessary, stirred.

2. A process according to claim 1, characterised in that the reaction takes place at temperatures of 80-140°C.

3. A process according to claims 1 and 2, characterised in that the reaction takes place at pressures of 1 to 5 MPa.

4. A process according to claims 1 to 3, characterised in that the volume ratio of aqueous to organic phase is 10 : 1 to 100 : 1.

5. A process according to claims 1 to 4, characterised in that the concentration of rhodium-containing complex compound in the aqueous phase is 0.8-12.0 mmol/kg of the aqueous phase.

**Revendications**

1. Procédé pour la fabrication d'aldéhydes par réaction d'oléfines avec le monoxyde de carbone et l'hydrogène en présence d'eau et d'un composé complexe soluble dans l'eau contenant du rhodium comme catalyseur sous pression et à température élevées, caractérisé en ce que l'on fait réagir des oléfines en $C_2$-$C_{20}$ avec une phase aqueuse qui contient par kg 0,1 à 15 mmol du complexe de rhodium soluble dans l'eau à une température de 20 à 160°C et sous une pression de 0,1 à 10 MPa, le rapport en volume de la phase aqueuse à la phase organique est de 1 : 100 à 100 : 1, on fait agir des ultrasons sur le mélange de réaction et éventuellement on agite.

2. Procédé selon la revendication 1, caractérisé en ce que la réaction est mise en oeuvre à des températures de 80 à 140°C.

3. Procédé selon les revendications 1 et 2, caractérisé en ce que la réaction est mise en oeuvre sous des pressions de 1 à 5 MPa.

4. Procédé selon les revendications 1 à 3, caractérisé en ce que le rapport en volume de la phase aqueuse et la phase organique est de 10 : 1 à 100 : 1.

5. Procédé selon les revendications 1 à 4, caractérisé en ce que la concentration du complexe contenant du rhodium dans la phase aqueuse est de 0,8-12,0 mmol par kg de phase aqueuse.